# EUROPEAN PATENT APPLICATION

(11) **EP 2 351 539 A2**
(43) Date of publication of application: **03.08.2011**
(21) Application number: 11165723.5
(22) Date of filing: 14.09.2006
(51) Int. Cl.: A61F 2/06, A61F 2/44, A61B 17/88

(54) **Methods and Devices for Stenting or Tamping a Fractured Vertebral Body**

(30) Priority: 29.09.2005 US 721619 P; 29.11.2005 US 289252
(62) Divisional of application: 06803639.1
(71) Applicant: Depuy Spine Inc., Raynham, MA 02767 (US)
(72) Inventor: Parsons, Matthew, Dartmouth, MA 02747 (US); O'Neil, Michael, West Barnstable, MA 02668 (US); Voellmicke, John, Cumberland, RI 02864 (US); Dooris, Andrew, Raynham, MA 02767 (US)
(74) Representative: Orr, Robert

(57) **Abstract**

Intravertebral bone stents and tamps are disclosed. In one embodiment, there is disclosed an intervertebral bone stent comprising: a reversibly expanding structure (135) containing multiple linkages (137) capable of transitioning the structure from a collapsed shape to an expanded shape, and an inner void (141); and a membrane (145) located within the inner void.

## Description

### BACKGROUND OF THE INVENTION

In vertebroplasty, the surgeon seeks to treat a compression fracture of a vertebral bodyby injecting bone cement such as PMMA into the fracture site. In one clinical report, Jensen et al., AJNR: 18 Nov. 1997, Jensen describes mixing two PMMA precursor components (one powder and one liquid) in a dish to produce a viscous bone cement; filling 10 ml syringes with this cement, injecting it into smaller 1 ml syringes, and finally delivering the mixture into the desired area of the vertebral body through needles attached to the smaller syringes.

U.S. Patent No. 5,108,404 ("Scholten") discloses inserting an inflatable device within a passage within the vertebral body, inflating the balloon to compact the cancellous bone and create an enlarged void, and finally injecting bone cement into the void. Scholten further dicloses inserting an irrigation nozzle into the vertebral body after removing the balloon and irrigating the void with normal saline. See column 7, lines 36-40. Scholten further discloses injecting the bone cement through a double-barreled injection gun having a cement delivery tube and an aspirating tube that aspirates constantly. See column 7, lines 42-50. US Published Patent Application 2002/0161373 ("Osorio") describes the percutaneous creation of a cavity (with a balloon catheter) within a vertebral bod and subsequent filling of the cavity with a bone filler. US Published Patent Application US 2002/0099384 ("Scribner") describes a two-chambered plunger device for driving a filler material into bone.

The patent literature reports many instances in which a stent is used to support an intervertebral disc space. For example, US Patent No. 6,395,034 ("Suddaby") describes an expandable stent as a prosthetic disc replacement that can be used with bone cement.
PCT Published Patent Application WO 01/10316 ("Ferree") describes devices for preventing the escape of material from a damaged disc annulus. The devices may include expandable, shape-memory or solidifying features. US Published Patent Application US 2002/0189622 ("Cauthen III") describes an expandable device for intervertebral disc reconstruction that is inserted into a disc annulus defect in a collapsed state and then expands (or is expanded) to occlude the defect.

US Patent No. 6,358,254 ("Anderson") describes a wedge-like stent implant for expanding a stenosed spinal canal.

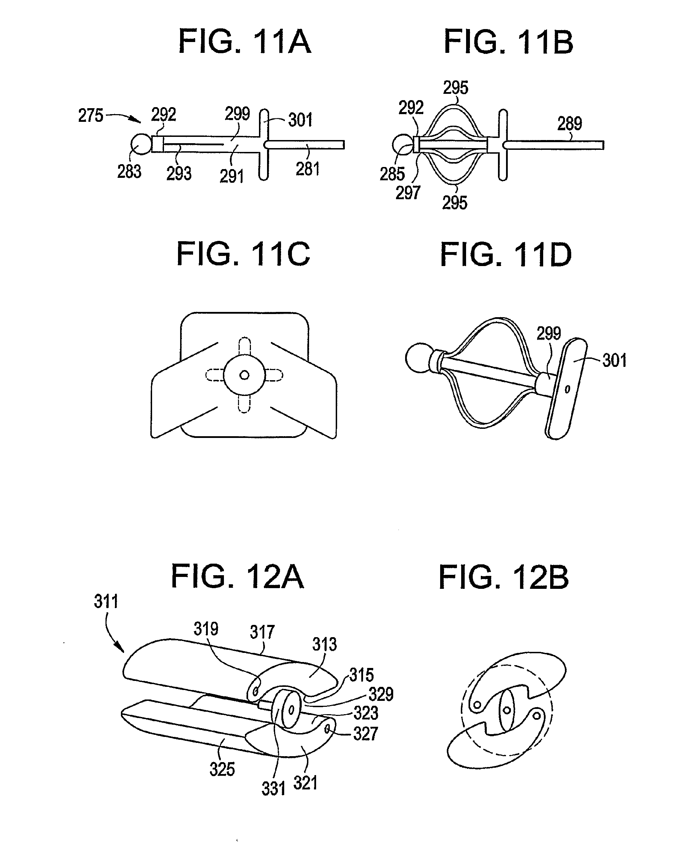

US Patent No. 6,679,886 ("Weikel") describes a memory metal bone tamp particularly adapted for vertebroplasty. See FIGS. 11A-D and 29 A-B. Weikel discloses that one tamp embodiment employs an expandable ring made from memory metal (such as superelastic nickel titanium alloy such as NITINOL^{™}, wherein the expandable ring has a preformed shape so that when the memory metal or NITINOL^{™} body is retracted into the body of the tamp, there is no expanded ring, and as the NITINOL^{™} body exits from the body of the tamp an expanding ring is formed.

PCT Published Patent Application WO 01/54598 ("Shavit") discloses an inflatable implant adapted to be anchored in the cancellous portion of a vertebral body, whereby the inflation of the anchor portion causes the implant to engage the cancellous bone.

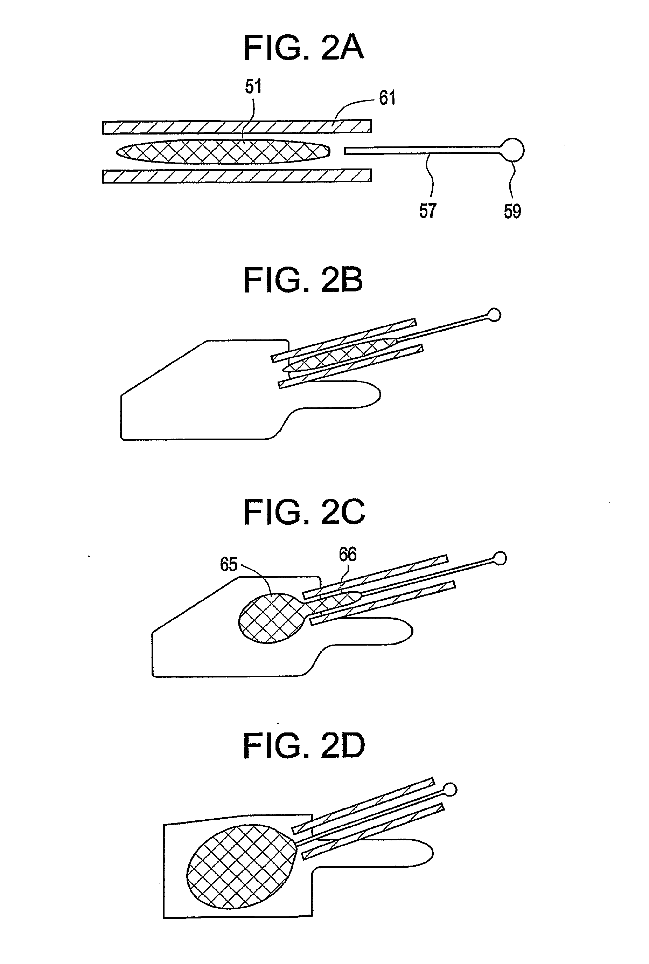

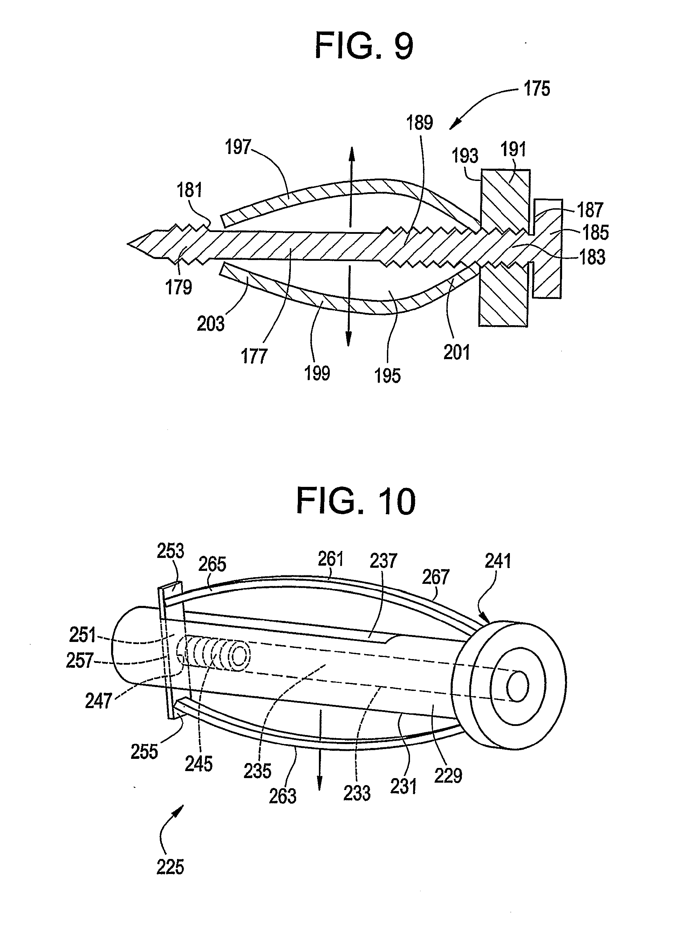

US Patent No. 6,127,597 ("Beyar") discloses systems for bone and spinal stabilization, fixation and repair, including intramedullar nails, intervertebral cages and prostheses designed for expansion from a small diameter for insertion into place to a larger diameter which stabilizes, fixes or repairs the bone. In one embodiment, Beyar discloses a memory metal stent adapted to engage the inner bone surface surrounding the intramedullary cavity to exert a strong outward radial force on the bone. See FIGS. 2A-2B of Beyar. In another embodiment, Beyar discloses memory metal bone stents made of a mesh geometry. See FIGS. 10A-D of Beyar. It appears that Beyar does not teach use of such as device as an intravertebral stent. See col. 29, lines 14-22 of Beyar.

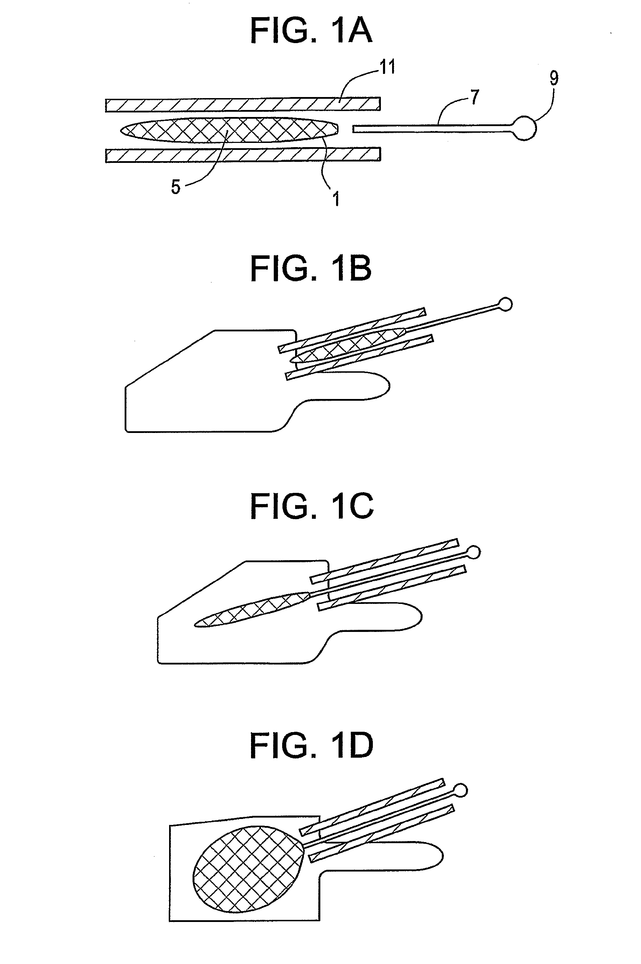

PCT Published Patent Application WO 00/44321 ("Globerman I") discloses an expandable element delivery system designed for delivering intervertebral fusion devices. In some embodiments, the expandable spacer is a tube having axial slits. When the spacer is axially axially compressed, the slits allow the formation of spikes. See FIGS. 1A-1D. PCT Published Patent Application WO 00/44319 ("Globerman II") discloses similar spacers and teaches that they may also be used as bone fixation devices. Globerman II discloses the use of such an expandable device for fixing a long bone. See FIG. 10A-B of Globerman II.

### SUMMARY OF THE INVENTION

In a first preferred embodiment of the present invention, there is provided an expandable intravertebral implant comprising memory metal.

Therefore, in accordance with the present invention, there is provided an intravertebral bone stent comprising a tubular member comprising a shape memory material.

Also in accordance with the present invention, there is provided a method of stabilizing a fracture vertebral body, comprising the steps of:
a) providing an intravertebral bone stent comprising a tubular member comprising a shape memory material in a collapsed state,
b) delivering the stent into the fractured vertebral body, and
c) expanding the stent to stabilize the fractured vertebral body.

In some embodiments thereof, the memory metal has a martinsitic M→ austentic A phase change between 22 °C and 37 °C. When the memory metal has such a characteristic, the stent can be made so that its martinsitic state describes a collapsed shape and its austentic state describes an expanded shape. Therefore, the stent can be delivered to the vertebral body in a collapsed, martinsitic state and in minimally invasive fashion and then undergo austenitic expansion upon body heating so that the stent creates a cavity within the vertebral body and stabilizes the fracture.

In some embodiments thereof, the memory metal has a superelastic property between the temperatures of 22 °C and 37 °C. The superelastic property allows the stent to withstand high stresses without experiencing plastic deformation or rupture. When the memory metal has such a superelastic characteristic, the stent can be deformed into a collapsed state and fit within a delivery cannula without plastic deformation or rupture. As the stent emerges from the cannula, it regains its original expanded shape so that the stent creates a cavity within the vertebral body and stabilizes the fracture.

In a second preferred embodiment of the present invention, there is provided an expandable intravertebral tamp comprising memory metal. The memory metal has a martinsitic M→ austentic A phase change between 22 °C and 37 °C. When the memory metal has such a characteristic, the tamp can be made so that its martinsitic state describes a collapsed shape and its austentic state describes an expanded shape. Therefore, the tamp can be delivered to the vertebral body in a collapsed, martinsitic state and in minimally invasive fashion and then undergo austenitic expansion upon body or active heating so that the tamp creates a cavity within the vertebral body and stabilizes the fracture.

Therefore, in accordance with the present invention, there is provided intravertebral bone tamp comprising:
a) a cannula having a throughbore, and
b) an expansion device diposed within the cannula, wherein the expansion device comprises a distal tubular member comprising a shape memory material having a martinsitic M→ austentic A phase change between 22 °C and 37 °C and a proximal rod.

Also in accordance with the present invention, there is provided a method of stabilizing a fractured vertebral body, comprising the steps of
a) providing an intravertebral bone tamp comprising a shape memory material having a martinsitic M→ austentic A phase change between 22 °C and 37 °C in a collapsed state,
b) delivering the tamp into the fractured vertebral body in the collapsed state, and heating the memory metal material to expand the tamp to stabilize the fractured vertebral body.

### DESCRIPTION OF THE FIGURES

FIGS. 1A-1F disclose the intravertebral delivery of a first memory metal stent of the present invention, wherein the stent expands upon body heating.
FIGS. 2A-2D disclose the intravertebral delivery of a second memory metal stent of the present invention, wherein the stent is superelastic and expands upon emergence from the cannula.
FIGS. 3A-3F disclose the intravertebral delivery of a memory metal tamp of the present invention, wherein the tamp expands upon body heating.
FIGS. 4A and 4B disclose a first embodiment of an expandable stent based upon a turnbuckle.
FIGS. 5A and 5B disclose a second embodiment of an expandable stent based upon a turnbuckle.
FIGS. 6A and 6B disclose a an expandable stent based upon a geodesic dome.
FIGS. 7A and 7B disclose a an expandable stent having an inner balloon.
FIGS. 8A and 8B disclose a first embodiment of a stent having rivet technology.
FIG. 9 discloses a second embodiment of a stent having rivet technology.
FIG. 10 discloses a third embodiment of a stent having rivet technology.
FIGS. 11A-D disclose a fourth embodiment of a stent having rivet technology.
FIGS. 12A and 12B disclose a stent based upon cam technology.

### DETAILED DESCRIPTION OF THE INVENTION

In other embodiments, the devices of the present invention are designed as implants (or "stents"), wherein the device is inserted into the vertebral body, expanded to create a cavity and stabilize the fracture, and then left within the vertebral body as a load-bearing or load-sharing implant. In these embodiments, any cavity created by the expansion of the device may be filled with a bone filler such as bone cement or bone growth agents. These stents are particularly useful when used in conjunction with bone growth agents, as they provide the required support for the fracture while the bone growth agents are forming bone.

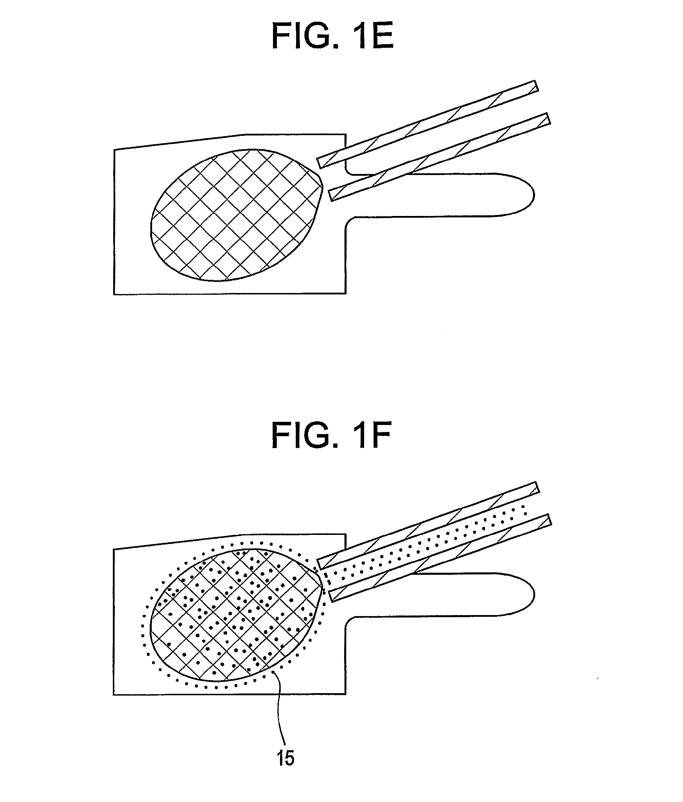

In some embodiments, the stent relies upon body heat to expand. This can occur when the memory metal possesses a martinsitic M→ austentic A phase change between 22 °C and 37 °C. Simply, the memory material is formed to have a first collapsed shape at a low temperature and a second expanded shape at a higher temperature.
- In these embodiments, and now referring to FIG. 1A, the stent 1 is provided within the throughbore of a cannula 11 in a collapsed form. The stent includes a tubular member 5 made of a memory material. In this particular embodiment, the distal tubular member is in the form of a mesh. Proximal to the tubular member within the cannula is a pusher rod 7 having a handle 9 at the proximal end thereof. Now referring to FIG. 1B, the distal ends of both the stent and cannula are inserted into the vertebral body while the stent is still in its collapsed form. Now referring to FIG. 1C, the handle of the pusher rod is advanced to push the stent into the vertebral body, while the cannula remains in place. Now referring to FIG. 1D, once the stent has been in the vertebral body for a sufficient period, the heat from the vertebral body (~37 °C) warms the memory material and induces a martensitic to austentic phase change in the stent, thereby causing the stent to expand and create a cavity. Now referring to FIG. 1E, the pusher rod is removed from the vertebral body and cannula. Optionally, and now referring to FIG. 1F, a flowable material 15 such as a bone cement or a bone growth agent is then injected into the cavity of the vertebral body through the cannula. The stent is then left within the vertebral body as an implant that supports the vertebral body. If the flowable agent is a bone cement, then the stent is essentially the load-sharing device that reduces the requirements on the cement. If the flowable agent is a bone growth agent, then the stent is essentially the load-bearing device during the early stages of bone formation.

In some embodiments, the device is provided outside the body at a temperature that imparts flexibility. In these embodiments, the device is provided in a cannula in a collapsed, flexible form. As the device is then inserted into the vertebral body, it expands to create a cavity. The device is then left within the vertebral body as a load-bearing or load-sharing implant.

In these embodiments, and now referring to FIG. 2A, the stent 51 is provided within the throughbore of a cannula 61 in a collapsed form. The stent includes a tubular member made of a memory material. In this particular embodiment, the distal tubular member is in the form of a mesh. Proximal to the tubular member within the cannula is a pusher rod 57 having a handle 59 at the proximal end thereof. Now referring to FIG. 2B, the distal ends of both the stent and cannula are inserted into the vertebral body while the stent is still in its collapsed form. Now referring to FIG. 2C, the handle of the pusher rod is advanced to push the distal portion 65 of the stent into the vertebral body, while the cannula remains in place. Because the stent is made of a superelastic material, the distal portion of the stent that has emerged from the cannula is no longer constrained by the cannula and so is able to expand to its unconstrained form. The proximal portion 66 of the stent that remains within the cannula is still in its constrained form. Now referring to FIG. 2D, once the entire stent has been advanced out of the cannula and into the vertebral body, for a sufficient period, the stent expands to stabilize the fracture. Next, as in FIGS. 1E and 1F, the pusher rod is removed from the vertebral body and cannula, and a flowable material such as a bone cement or a bone growth agent is then injected into the cavity of the vertebral body through the cannula. The stent is then left within the vertebral body as an implant that supports the vertebral body.

In some embodiments, the devices of the present invention are designed as tamps, wherein the device is inserted into the vertebral body, expanded to create a cavity and then withdrawn from the vertebral body. In these embodiments, the cavity created by the expansion of the device is then filled with a bone filler such as bone cement or bone growth agents.

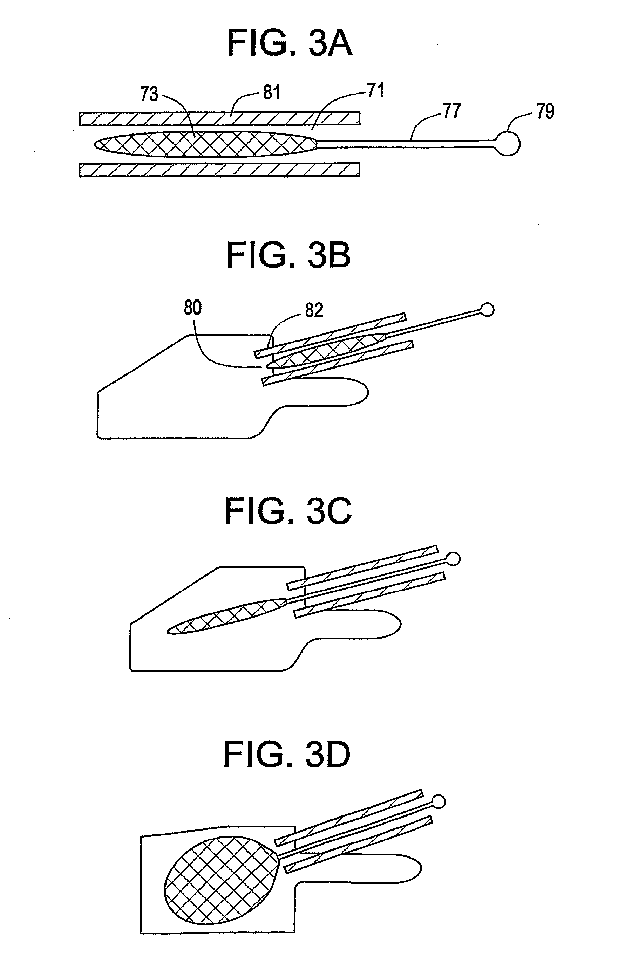

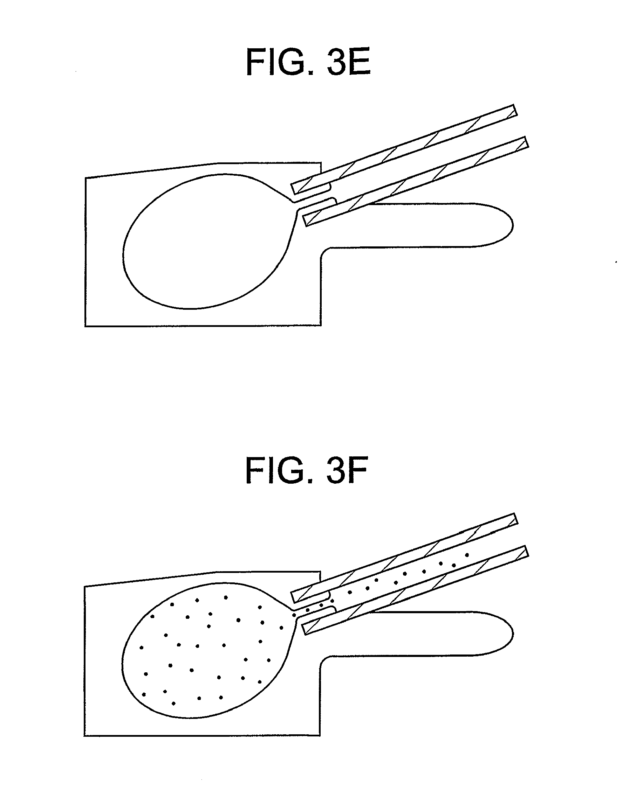

In these embodiments, and now referring to FIG. 3A, the tamp 71 is provided within the throughbore of a cannula 81 in a collapsed form. The stent includes a distal expansion device 73 made of a memory material attached to a proximal pusher rod 77 having a handle 79 at the proximal end thereof Now referring to FIG. 3B, the distal ends 80, 82 of both the tamp and cannula are inserted into the vertebral body while the expansion device is still in its collapsed form. Now referring to FIG. 3C, the handle of the pusher rod is advanced to push the expansion device into the vertebral body, while the cannula remains in place. Now referring to FIG. 3D, once the distal expansion device has been in the vertebral body for a sufficient period, heat from the vertebral body (~37 °C) warms the memory material and induces a martensitic to austentic phase change in the expansion device, thereby causing the expansion device to expand and create a cavity. Now referring to FIG. 3E, the tamp is removed, thereby leaving a cavity. Now referring to FIG. 3F, a flowable material 15 such as a bone cement or a bone growth agent is then injected into the cavity of the vertebral body through the cannula.

The devices of the present invention can be made from conventional structural shape memory biomaterials such as metals or polymers. In terms of shape-memory metals, those materials set forth in U.S. Pat. No. 5,954,725, the entire contents of which are incorporated herein by reference, may be used, including, but not limited to alloys of copper and zinc, nickel titanium, silver and cadmium, and other metals and materials, including Nitinol.

For the purposes of the present invention, the terms "bone-forming agent" and "bone growth agent" are used interchangeably. Typically, the bone-forming agent may be:
a) a growth factor (such as an osteoinductive or angiogenic factor),
b) osteoconductive (such as a porous matrix of granules),
c) osteogenic (such as viable osteoprogenitor cells), or
d) plasmid DNA.

In some embodiments, the formulation comprises a liquid, solid or gelled carrier, and the bone forming agent is soluble in the carrier.

In some embodiments, the bone forming agent is a growth factor. As used herein, the term "growth factor" encompasses any cellular product that modulates the growth or differentiation of other cells, particularly connective tissue progenitor cells. The growth factors that may be used in accordance with the present invention include, but are not limited to, members of the fibroblast growth factor family, including acidic and basic fibroblast growth factor (FGF-1 and FGF-2) and FGF-4; members of the platelet-derived growth factor (PDGF) family, including PDGF-AB, PDGF-BB and PDGF-AA; EGFs; VEGF; members of the insulin-like growth factor (IGF) family, including IGF-I and -II; the TGF-β superfamily, including TGF-β1, 2 and 3; osteoid-inducing factor (OIF), angiogenin(s); endothelins; hepatocyte growth factor and keratinocyte growth factor; members of the bone morphogenetic proteins (BMPs) BMP-1, BMP-3, BMP-2, OP-1, BMP-2A, BMP-2B, BMP-7 and BMP-14, including MP-52; BBGF-1 and HBGF-2; growth differentiation factors (GDFs), including GDF-5, members of the hedgehog family of proteins, including indian, sonic and desert hedgehog; ADMP-1; bone-forming members of the interleukin (IL) family; GDF-5; and members of the colony-stimulating factor (CSF) family, including CSF-1, G-CSF, and GM-CSF; and isoforms thereof.

In some embodiments, the growth factor is selected from the group consisting of TGF-β, bFGF, and IGF-1. These growth factors are believed to promote the regeneration of bone. In some embodiments, the growth factor is TGF-β. More preferably, TGF-β is administered in an amount of between about 10 ng/ml and about 5000 ng/ml, for example, between about 50 ng/ml and about 500 ng/ml, e.g., between about 100 ng/ml and about 300 ng/ml.

In some embodiments, platelet concentrate is provided as the bone forming agent. In one embodiment, the growth factors released by the platelets are present in an amount at least two-fold (*e.g*., four-fold) greater than the amount found in the blood from which the platelets were taken. In some embodiments, the platelet concentrate is autologous. In some embodiments, the platelet concentrate is platelet rich plasma (PRP). PRP is advantageous because it contains growth factors that can restimulate the growth of the bone, and because its fibrin matrix provides a suitable scaffold for new tissue growth.

In some embodiments, the bone forming agent comprises an effective amount of a bone morphogenic protein (BMP). BMPs beneficially increasing bone formation by promoting the differentiation of mesenchymal stem cells (MSCs) into osteoblasts and their proliferation.

In some embodiments, between about 1 ng and about 10 mg of BMP are intraosseously administered into the target bone. In some embodiments, between about 1 microgram (µg) and about 1 mg of BMP are intraosseously administered into the target bone.

In some embodiments, the bone forming agent comprises an effective amount of a fibroblast growth factor (FGF). FGF is a potent mitogen and is angiogenic, and so attracts mesenchymal stem cells to the target area. It is further believed that FGF stimulates osteoblasts to differentiate into osteocytes.

In some embodiments, the FGF is acidic FGF (aFGF).

In some embodiments, the FGF is basic FGF (bFGF).

In some embodiments, between about 1 microgram (µg) and about 10,000 µg of FGF are intraosseously administered into the target bone. In some embodiments, between about 10 µg and about 1,000 µg of FGF are intraosseously administered into the target bone. In some embodiments, between about 50 µg and about 600 µg of FGF are intraosseously administered into the target bone.

In some embodiments, between about 0.1 and about 4 mg/kg/day of FGF are intraosseously administered into the target bone. In some embodiments, between about 1 and about 2 mg/kg/day of FGF are intraosseously administered into the target bone.

In some embodiments, FGF is intraosseously administered into the target bone in a concentration of between about 0.1 mg/ml and about 100 mg/ml. In some embodiments, FGF is intraosseously administered into the target bone in a concentration of between about 0.5 mg/ml and about 30 mg/ml. In some embodiments, FGF is intraosseously administered into the target bone in a concentration of between about 1 mg/ml and about 10 mg/ml.

In some embodiments, FGF is intraosseously administered into the target bone in an amount to provide a local tissue concentration of between about 0.1 mg/kg and about 10 mg/kg.

In some embodiments, the formulation comprises a hyaluronic acid carrier and bFGF. In some embodiments, formulations described in U.S. Patent No. 5,942,499 ("Orquest") are selected as FGF-containing formulations.

In some embodiments, the bone forming agent comprises an effective amount of insulin-like growth factor. IGFs beneficially increase bone formation by promoting mitogenic activity and/or cell proliferation.

In some embodiments, the bone forming agent comprises an effective amount of parathyroid hormone (PTH). Without wishing to be tied to a theory, it is believed that PTH beneficially increases bone formation by mediating the proliferation of osteoblasts.

In some embodiments, the PTH is a fragment or variant, such as those taught in U.S. Patent Nos. 5,510,370 (Hock) and 6,590,081 (Zhang), and published patent application 2002/0107200 (Chang), the entire contents of which are incorporated herein in their entirety. In one embodiment, the PTH is PTH (1-34) (teriparatide), *e.g*., FORTEO^{®} (Eli Lilly and Company). In some embodiments, the BFA is a parathyroid hormone derivative, such as a parathyroid hormone mutein. Examples of parathyroid muteins are discussed in U.S. Patent No. 5,856,138 (Fukuda), the entire contents of which are incorporated herein in its entirety.

In some embodiments, the bone forming agent comprises an effective amount of a statin. Without wishing to be tied to a theory, it is believed that statins beneficially increase bone formation by enhancing the expression of BMPs.

In some embodiments, the bone forming agent is a porous matrix, and is preferably injectable. In some embodiments, the porous matrix is a mineral. In one embodiment, this mineral comprises calcium and phosphorus. In some embodiments, the mineral is selected from the group consisting of calcium phosphate, tricalcium phosphate and hydroxyapatite. In one embodiment, the average porosity of the matrix is between about 20 and about 500 µm, for example, between about 50 and about 250 µm. In yet other embodiments of the present invention, *in situ* porosity is produced in the injected matrix to produce a porous scaffold in the injected fracture stabilizing cement. Once the *in situ* porosity is produced in the target tissue, the surgeon can inject other therapeutic compounds into the porosity, thereby treating the surrounding tissues and enhancing the remodeling process of the target tissue and the injectable cement.

In some embodiments, the mineral is administered in a granule form. It is believed that the administration of granular minerals promotes the formation of the bone growth around the minerals such that osteointegration occurs.

In some embodiments, the mineral is administered in a settable-paste form. In this condition, the paste sets up *in vivo,* and thereby immediately imparts posttreatment mechanical support to the fragile osteoporotic body.

In another embodiment, the treatment is delivered via injectable absorbable or non-absorbable cement to the target tissue. The treatment is formulated using bioabsorbable macro-sphere technologies, such that it will allow the release of the bone forming agent first, followed by the release of the anti-resorptive agent. The cement will provide the initial stability required to treat pain in fractured target tissues. These tissues include, but are not limited to, hips, knee, vertebral body fractures and iliac crest fractures. In some embodiments, the cement is selected from the group consisting of calcium phosphate, tricalcium phosphate and hydroxyapatite. In other embodiments, the cement is any hard biocompatible cement, including PMMA, processed autogenous and allograft bone. Hydroxylapatite is a preferred cement because of its strength and biological profile. Tricalcium phosphate may also be used alone or in combination with hydroxylapatite, particularly if some degree of resorption is desired in the cement.

In some embodiments, the porous matrix comprises a resorbable polymeric material.

In some embodiments, the bone forming agent comprises an injectable precursor fluid that produces the *in situ* formation of a mineralized collagen composite. In some embodiments, the injectable precursor fluid comprises:
a) a first formulation comprising an acid-soluble type I collagen solution (preferably between about 1 mg/ml and about 7 mg/ml collagen) and
b) a second formulation comprising liposomes containing calcium and phosphate.

Combining the acid-soluble collagen solution with the calcium- and phosphate-loaded liposomes results in a liposome/collagen precursor fluid, which, when heated from room temperature to 37 °C, forms a mineralized collagen gel.

In some embodiments, the liposomes are loaded with dipalmitoylphosphatidylcholine (90 mol%) and dimyristoyl phosphatidylcholine (10 mol%). These liposomes are stable at room temperature but form calcium phosphate mineral when heated above 35°C, a consequence of the release of entrapped salts at the lipid chain melting transition. One such technology is disclosed in Pederson, Biomaterials 24: 4881-4890 (2003), the specification of which is incorporated herein by reference in its entirety.

Alternatively, the *in situ* mineralization of collagen could be achieved by an increase in temperature achieved by other types of reactions including, but not limited to, chemical, enzymatic, magnetic, electric, photo- or nuclear. Suitable sources thereof include light, chemical reaction, enzymatically controlled reaction and an electric wire embedded in the material. To further elucidate the electric wire approach, a wire (which can be the reinforcement rod) can first be embedded in the space, heated to create the calcium deposition, and then withdrawn. In some embodiments, this wire may be a shape memory such as nitinol that can form the shape. Alternatively, an electrically-conducting polymer can be selected as the temperature raising element. This polymer is heated to form the collagen, and is then subject to disintegration and resorption *in situ,* thereby providing space adjacent the mineralized collagen for the bone to form.

In one embodiment, the bone forming agent is a plurality of viable osteoprogenitor cells. Such viable cells, introduced into the bone, have the capability of at least partially repairing any bone loss experienced by the bone during the osteoporotic process. In some embodiments, these cells are introduced into the cancellous portion of the bone and ultimately produce new cancellous bone. In others, these cells are introduced into the cortical region and produce new cortical bone.

In some embodiments, these cells are obtained from another human individual (allograft), while in other embodiments, the cells are obtained from the same individual (autograft). In some embodiments, the cells are taken from bone tissue, while in others, the cells are taken from a non-bone tissue (and may, for example, be mesenchymal stem cells, chondrocytes or fibroblasts). In others, autograft osteocytes (such as from the knee, hip, shoulder, finger or ear) may be used.

In one embodiment, when viable cells are selected as an additional therapeutic agent or substance, the viable cells comprise mesenchymal stem cells (MSCs). MSCs provide a special advantage for administration into an uncoupled resorbing bone because it is believed that they can more readily survive the relatively harsh environment present in the uncoupled resorbing bone; that they have a desirable level of plasticity; and that they have the ability to proliferate and differentiate into the desired cells.

In some embodiments, the mesenchymal stem cells are obtained from bone marrow, such as autologous bone marrow. In others, the mesenchymal stem cells are obtained from adipose tissue, preferably autologous adipose tissue.

In some embodiments, the mesenchymal stem cells injected into the bone are provided in an unconcentrated form, *e.g*., from fresh bone marrow. In others, they are provided in a concentrated form. When provided in concentrated form, they can be uncultured. Uncultured, concentrated MSCs can be readily obtained by centrifugation, filtration, or immuno-absorption. When filtration is selected, the methods disclosed in U.S. Patent No. 6,049,026 ("Muschler"), the specification of which is incorporated herein by reference in its entirety, can be used. In some embodiments, the matrix used to filter and concentrate the MSCs is also administered into the uncoupled resorbing bone.

In some embodiments, bone cells (which may be from either an allogenic or an autologous source) or mesenchymal stem cells, may be genetically modified to produce an osteoinductive bone anabolic agent which could be chosen from the list of growth factors named herein. The production of these osteopromotive agents may lead to bone growth.

In some embodiments, the osteoconductive material comprises calcium and phosphorus. In some embodiments, the osteoconductive material comprises hydroxyapatite. In some embodiments, the osteoconductive material comprises collagen. In some embodiments, the osteoconductive material is in a particulate form.

Recent work has shown that plasmid DNA will not elicit an inflammatory response as does the use of viral vectors. Genes encoding bone (anabolic) agents such as BMP maybe efficacious if injected into the uncoupled resorbing bone. In addition, overexpression of any of the growth factors provided herein or other agents which would limit local osteoclast activity would have positive effects on bone growth. In one embodiment, the plasmid contains the genetic code for human TGF-β or erythropoietin (EPO).

Accordingly, in some embodiments, the additional therapeutic agent is selected from the group consisting of viable cells and plasmid DNA.

The above discussion has focused upon the use of a singular implant to create a large void in the cancellous bone, but an alternative embodiment using multiple, smaller sized implants placed in series could also be effective. These smaller, memory metal structures, could be of various shapes (e.g., spherical, football, cylinder, coil, ellipsoid, crumpled ball of wire). They are sequentially inserted in a collapsed state and then expanded (either through heat activated phase transformation or through superelastic deformation) to locally compact tissue to create a network of small voids in the vertebral body. This is an improvement over the prior art which describes the insertion of solid metal beads or disks to expand the vertebral body. The space created with expanding memory metal implants is porous and can receive a bone cement or other injectable biomaterial to create a composite structure. A porous structure could also allow for bony ingrowth for a better bone/implant interface.

Therefore, in accordance with the present invention, there is provided a method of stabilizing a fractured vertebral body, comprising the steps of:
a) providing a plurality of implants comprising a shape memory material in a collapsed state,
b) delivering the plurality of implants through a cannula into the fractured vertebral body, and
c) expanding the plurality of implants to an expanded state to stabilize the fractured vertebral body.

Some methods appropriate with this technique may include, for example, sequentially placing the implants, waiting for body temperature to heat and expand the memory metal structures, lavaging blood and marrow from porous network of metal, and filling the voids with bone cement or other biologic agent.

In one embodiment of the present invention, the bone stent incorporates a collapsible structure containing multiple linkages that can transition the stent from a minimal volume to a maximum volume. Preferably, the collapsible structure is a Hoberman sphere. However, the shape of the multiple-linkage stent is not limited to a sphere: domes (hemispheres), arches, cylinders, and combinations thereof may also be used.

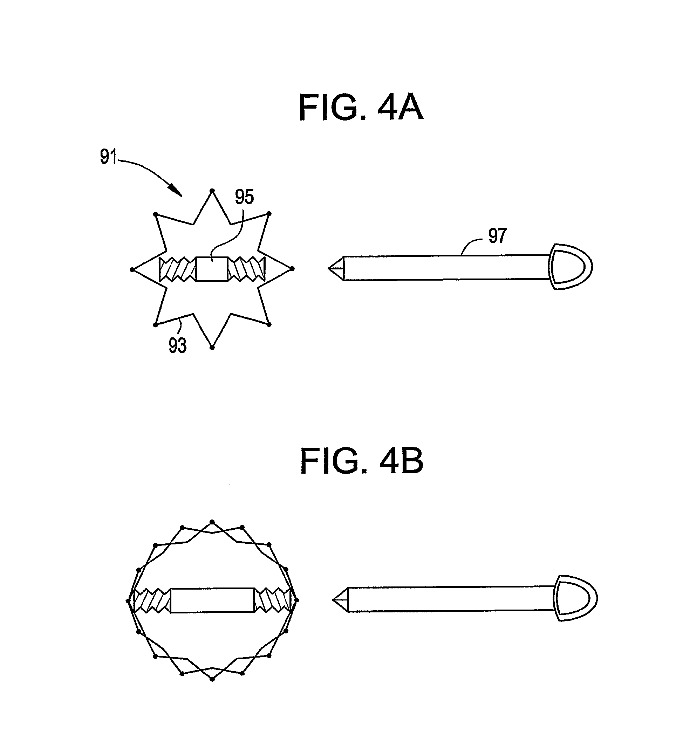

Now referring to FIG. 4A, in one multiple linkage embodiment, a spherical construct 91 of linked struts 93 could be actuated with a turnbuckle 95 or similar mechanism to transition from a minimally invasively inserted collapsed sphere to an expanded sphere. Now referring to FIG. 4B, the turnbuckle could be actuated remotely, or with a simple torque applicator (e.g., screwdriver 97), that would drive apart opposing ends of the sphere, thereby driving expansion of the entire stent. The turnbuckle would then prevent collapse of the stent, allowing it to bear load. Clips or crimps could be used to provide additional securement of the struts.

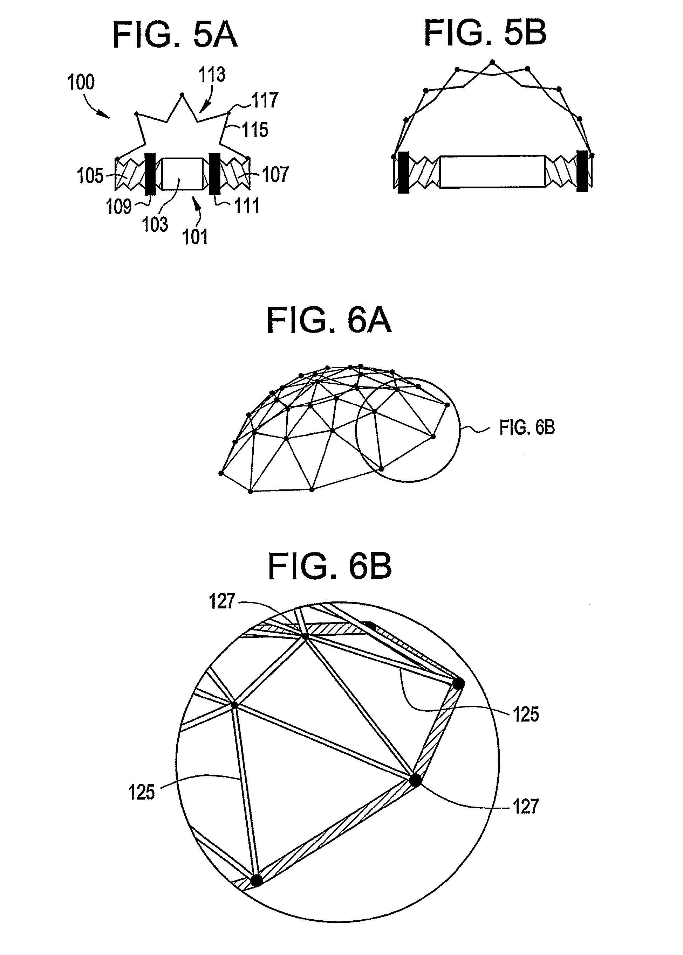

Now referring to FIG. 5A, there is provided an intravertebral stent 100, comprising:
a) a turnbuckle 101 comprising a shaft 103 having a first threaded end portion 105 and a second oppositely threaded end portion 107,
b) a first nut 109 threadably received upon the first threaded end portion,
c) a second nut 111 threadably received upon the second oppositely threaded end portion,
d) an expandable structure 113 comprising a plurality of struts 115 and means 117 for connecting the struts in a cooperative pattern, the struts including a first and second end struts,
wherein the first end strut bears against the first nut and the second end strut bears against the second nut.

Now referring to FIG. 5B, actuation of the turnbuckle forces the nuts to move to their respective ends, thereby expanding the expandable structure.

In some embodiments, the expandable structure is geodesic structure. In the present invention, geodesic structures comprise structural support members and a means for connecting the support members to one another. In some embodiments, and now referring to FIGS. 6A and 6B, the geodesic structures are geodesic domes, and include a plurality of strut members 125 which make up the dome itself, and means 127 for connecting the strut members to one another in the appropriate pattern to produce the desired dome structure.

The connecting means 127 of the geodesic structures may include hubs which comprise hollow, cylindrically-shaped tubular lengths, which are provided with means adaptive for connection of the strut members in a cooperative pattern. The hubs have locations spaced radially about their outside surfaces whereupon the struts are to be fastened. One example of a connecting means so suited is described in U.S. Pat. No. 4,521,998 and comprises a hinge plate. Another connecting means is described in U.S. Pat. No. 4,203,265 which comprises a hub and strut. U.S. Pat. No. 4,194,851 discloses a universal hub for geodesic domes which comprises a wing nut and two metal plates. Other systems for connecting the strut members of geodesic domes to one another are described in U.S. Pat. Nos.: 3,908,975; 4,531,333; 4,901,483; 4,511,278; 4,236,473; 5,165,207; 4,308,698; 4,365,910; 4,905,443; 4,319,853; and 4,464,073, the specifications of which are incorporated by reference in their entireties.

The struts 125 are generally shaped in the form of a rectangular solid, and are equipped with at least one threaded screw-type fastener having one end protruding from an end portion of the strut. The strut members may be constructed from materials which include metal and polymeric composites. The hubs may have a plurality of specially-shaped slotted holes on their surface which allow for the insertion of the threaded fastener portions of the struts through the holes, and a lateral motion of the strut with respect to the hub in order to locate the struts into their desired positions. Into the ends of the strut members are cut either a v-shaped or circular groove coincident with the width dimension of the strut for increased structural integrity of the joint formed, which effectively stabilizes the strut with respect to the cylindrical surface of the hub to provide a synergistic locking effect. The link between a strut member and the hub is completed by either tightening a nut as in the case of when the threaded fastener is a bolt, or by simple clockwise rotation of a large screw when such is employed. The struts could be formed from any number of materials, including polymers, composites, metals, resorbable materials, or combinations thereof.

In some embodiments, the multiple linkage stents are reversibly expanding structures. Such reversibly expanding structures may be made in accordance with US Patent Nos. 4,942,700 ("Hoberman I"), and 6,219,974 ("Hoberman II"), the specifications of which are incorporated by reference in their entireties.

In some embodiments, the reversibly expanding structures maintain an overall curved geometry as they expand or collapse in a synchronized manner. Structures of this kind are comprised of special mechanisms hereinafter referred to as "loop-assemblies". These assemblies are in part comprised of angulated strut elements that have been pivotally joined to other similar elements to form scissors-pairs. These scissors-pairs are in turn pivotally joined to other similar pairs or to hub elements forming a closed loop. When this loop is folded and unfolded, certain critical angles are constant and unchanging. These unchanging angles allow for the overall geometry of structure to remain constant as it expands or collapses.

In some embodiments, the reversibly expandable structures are formed from loop assemblies comprising interconnected pairs of polygonal shaped links. Each loop assembly preferably has polygon links with at least three pivot joints and at least some of the polygon links have more than three pivot joints. Additionally, these links lie essentially on the surface of the structure or parallel to the plane of the surface of the structure. Each polygon link has a center pivot joint for connecting to another link to form a link pair. Each link also has at least one internal pivot joint and one perimeter pivot joint. The internal pivot joints are used for connecting link pairs to adjacent link pairs to form a loop assembly. Loop assemblies can be joined together and/or to other link pairs through the perimeter pivot joints to form structures. In one embodiment, link pairs may be connected to adjacent link pairs in a loop assembly through hub elements that are connected at the respective internal pivot joints of the two link pairs. Similarly hub elements can be used to connect loop assemblies together or loop assemblies to other link pairs through the perimeter pivot joints. In yet another embodiment, the pivot joints can be designed as living hinges if constructed from appropriate flexible materials such as polypropyilene or nitinol.

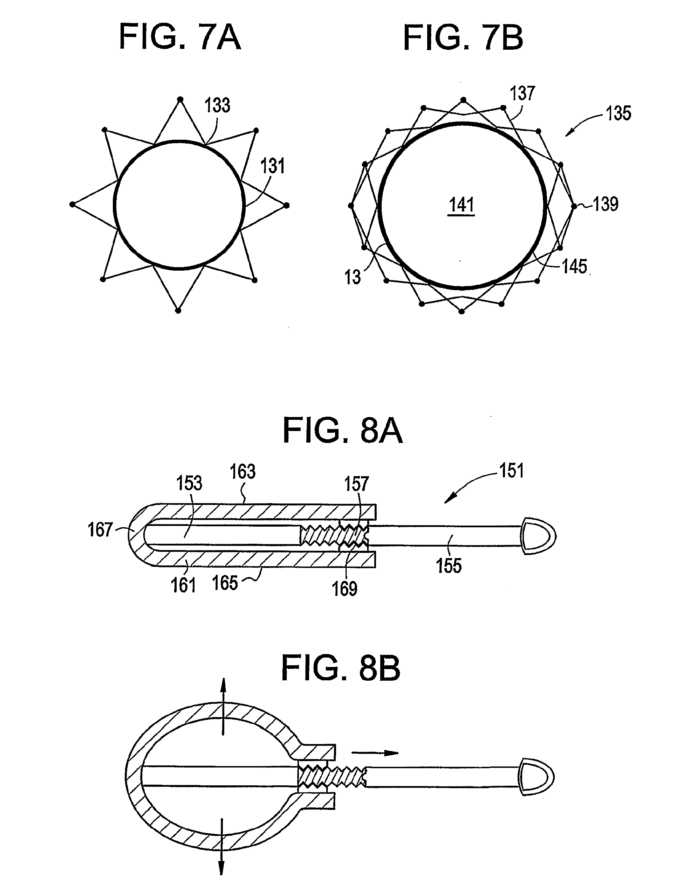

In some embodiments, the stent could be coupled with a compliant sheet or fabric. This fabric could be in the form of a membrane, such as a balloon, that would expand the struts or stent from a closed position to an open position. For example, and now referring to FIG. 7A and 7B, the turnbuckle of FIG. 4A could be replaced with a collapsed membrane131 whose outer surface is attached to the inner links 133 of the multiple-linkage stent. Now referring to FIG. 7B, upon expansion of the membrane(through, for example, the introduction of a sufficient amount of fluid into the balloon), the stent is forced from its collapsed state to its expanded state. Alternatively, the stent could be driven open, as previously described, thereby holding the fabric in a state of maximum volume, and enabling the void inside the fabric to be filled with a bone growth agent.

Therefore, in accordance with the present invention, there is provided a stent comprising:
a) an expandable structure 135 comprising a plurality of strut members 137 and means 139 for connecting the strut members to allow transition the of structure from a minimal volume to a maximum volume, the expandable structure having an inner void 141, and
b) a membrane145 located within the inner void.

Furthermore, the expanded membranecould be used to hold the stent open as a permanent part of the implant. Alternatively, the fabric could be biodegradable, so as to allow timed release of its contents, which might include osteoinductive/conductive/genic agents, or anti-biotic/septic agents.

Alternatively, the balloon's inner surface could be connected to the outer links of the multiple-linkage stent.

Now referring to FIG. 8A, in another embodiment, the stent could function in a manner similar to a rivet. The stent could comprise:
a) a rod 151 having a distal end portion 153, a proximal end portion 155 and a threaded intermediate portion 157, and
b) a deformable shell 161 having an upper wall 163, a lower wall 165, a distal intermediate wall 167 located between the upper and lower walls, and a proximal threaded lumen 169,
wherein the distal end portion of the rod is attached to the intermediate wall of the deformable shell, and
wherein the threaded intermediate portion of the rod is received in the threaded lumen.

The stent of FIG. 8A could be placed inside the bone by simply pushing the rod distally. Now referring to FIG. 8B, upon appropriate rotation of the rod, the rod will be drawn proximally, thereby causing the proximal and distal portions of the shell to be compressed towards each other, and causing expansion of the upper and lower walls, like a rivet. This expanded space can then be filled with a bone growth agent.

Now referring to FIG. 9, in another embodiment based upon rivet technology, the stent 175 could comprise:
a) a rod 177 having a distal end portion 179 forming a proximal shoulder 181, a proximal end portion 183 having an enlarged head 185 forming a distal shoulder 187, and a threaded intermediate shaft portion 189;
b) a threaded nut 191 having a distal face 193, the nut threadably received upon the threaded intermediate shaft portion of the rod; and
c) a deformable shell 195 having an upper wall 197 and a lower wall 199, each wall having a proximal end 201 and a distal end 203,
wherein the proximal end portion of each wall of the deformable shell bears against the distal face of the nut, and
wherein the distal end portion of each wall of the deformable shell bears against the proximal shoulder of the rod.

The walls of the deformable shell are constrained to be between the moveable nut and the proximal shoulder of the distal end portion of the rod. As the nut of FIG. 9 is advanced distally along the shaft of the rod, the walls of the deformable shell compress and bulge outward. This outward motion forms the desired space within the vertebral body that can then be filled with a flowable agent.

Now referring to FIG. 10, in another embodiment based upon rivet technology, the stent 225 could comprise:
a) a tube 229 having an outer surface 231, and inner threaded surface 233, a throughbore 235, and upper 237 and lower (not shown) slots extending from the outer surface to the throughbore, and a distal end shoulder 241 radially extending from the outer surface;
b) a threaded nut 245 having a distal face, the nut threadably received upon the threaded inner surface of the tube;
c) a plate 251 having an upper end portion 253, a lower end portion 255, and an intermediate portion 257, the upper end of the plate extending from the upper slot and the lower end of the plate extending from the lower slot and
d) deformable upper 261 and lower 263 walls, each wall having a proximal end 265 and a distal end 267,
wherein the distal face of the threaded nut abuts the intermediate portion of the plate, wherein the proximal end portion of the upper wall abuts (and is preferably attached to) the upper end portion of the plate,
wherein the proximal end portion of the lower wall abuts (and is preferably attached to) the lower end portion of the plate,
wherein the distal end portion of each wall abuts the distal end shoulder.

The walls are constrained to be between the moveable plate and the distal end shoulder. As the nut of FIG. 10 is advanced towards the distal face along the threaded ID of the tube, it pushes the moveable plate ahead of it, thereby causing the walls attached thereto to compress and bulge outward (as shown by arrow). This outward motion forms the desired space within the vertebral body that can then be filled with a flowable agent.

Now referring to FIGS. 11A and 11B, in another embodiment based upon rivet technology, the stent 275 could comprise:
a) a rod 281 having a distal end portion 283 forming a proximal shoulder 285, an intermediate portion (not shown), and a proximal end portion 289,
b) a tube 291 received upon the rod, the tube having an unslitted distal end 292, plurality of intermediate longitudinal slits 293 forming a plurality of collapsible walls 295 having a distal end 297, and unslitted proximal portion 299 having a proximal flange 301;
wherein the distal end portion of the rod extends from the tube, and
wherein the unslitted distal end of the tube bears against the proximal shoulder of the distal end portion of the rod.

Now referring to FIG. 11B, when the proximal end portion of the rod is pulled proximally, the proximal shoulder 285 bears against the distal end of the tube, forcing compression of the collapsible walls.
FIG. 11C shows the stent of FIG. 11B implanted within a vertebral body.
FIG. 11D shows the stent wherein the proximal end portion of the rod has been removed after expansion. Preferably, unslitted proximal portion 299 of the tube is a sufficient length to traverse the pedicle into which the stent has been placed.

Now referring to FIG. 12A, in some embodiments of the present invention, the intervertebral bone stent 31 includes a cam and comprises:
a) a first hemi-tube 313 having an inside surface 315, an outside surface 317 and a first longitudinal hinge 319,
b) a second hemi-tube 321 having an inside surface 323, an outside surface 325 and a second longitudinal hinge 327, the inside surface of the second hemi-tube opposing the inside surface of the first hemi-tube to form an inner bore 329 between the two hemi-tubes,
c) a substantially oval cam 331 located within the inner bore.

The stent of FIG. 12A is inserted into the vertebral body in its collapsed state, with the oval cam oriented so that the outer surfaces of its minor axis abut the inside surfaces of the hemi-tubes. Now referring to FIG. 12B, when the cam is rotated about 90 degrees, the cam is now oriented so that the outer surfaces of its major axis abut the inside surfaces of the hemi-tubes, thereby spreading the two hemi-tubes apart to compact the adjacent bone. Rotating the cam back to its original position brings the hemi-tubes back to their original positions, thereby leaving voids in the regions into which the hemi-tubes moved during the first rotation of the cam.

In reference to methods for holding, introducing and dispensing the stents into the vertebral bodies, the devices for stenting or tamping of fractured vertebral bodies can be either:
a) pushed by a simple plunger that is slideably advanced within a cannula, threadably advanced, lever action advanced, or spring advanced until targeted treamement location is reached, or
b) attached to a plunger element by press fit, snap fit, threaded, keyed, or snap ring, and remotely released at targeted treamement location is reached.

In a further aspect of the invention, there is provided a method of stabilizing a fracture vertebral body, comprising the steps of:
a) providing an intravertebral bone stent comprising a tubular member comprising a shape memory material in a collapsed state,
b) delivering the stent into the fractured vertebral body, and
c) expanding the stent to stabilize the fractured vertebral body.

The shape memory material may have a martinsitic M→ austentic A phase change between 22 °C and 37 °C, and the expansion of the stent occurs upon body heating. The shape memory material may have a superelastic characteristic between 22 °C and 37 °C, the stent may be delivered through a cannula, and the expansion of the stent may occur as the stent emerges from the cannula. The tubular member may be a mesh. The shape memory material may be selected from the group consisting of a metal and a polymer. The expansion of the stent may create a cavity, and the method may further comprise the steps of: d) flowing a flowable material into the cavity. The flowable material may be selected from the group consisting of a bone cement and a bone growth agent. The flowable material may be a bone growth agent. The bone growth agent may comprise a growth factor. The bone growth agent may comprise a porous matrix. The bone growth agent may comprise viable cells.

In a further aspect of the invention, there is provided an intravertebral bone tamp comprising:
a) a cannula having a throughbore, and
b) an expansion device diposed within the cannula, wherein the expansion device comprises a distal tubular member comprising a shape memory material having a martinsitic M→ austentic A phase change between 22 °C and 37 °C and a proximal rod.

The tubular member may be a mesh. The tubular member may be solid. The shape memory material may be selected from the group consisting of a metal and a polymer.

In a further aspect of the invention, there is provided a method of stabilizing a fractured vertebral body, comprising the steps of
a) providing an intravertebral bone tamp comprising a shape memory material having a martinsitic M→ austentic A phase change between 22 °C and 37 °C in a collapsed state,
b) delivering the tamp into the fractured vertebral body in the collapsed state, and
c) heating the memory metal material to expand the tamp to stabilize the fractured vertebral body.

The tamp may have a distal tubular member having a mesh shape. The shape memory material may be selected from the group consisting of a metal and a polymer. The expansion of the tamp may create a cavity, and may further comprise the steps of: d) flowing a flowable material into the cavity. The flowable material may be selected from the group consisting of a bone cement and a bone growth agent. The flowable material may be a bone growth agent. The bone growth agent may comprise a growth factor. The bone growth agent may comprise a porous matrix. The bone growth agent may comprise viable cells. The method may further comprise the steps of: d) removing the tamp from the vertebral body.

In a further aspect of the invention, there is provided a method of stabilizing a fracture vertebral body, comprising the steps of:
a) providing a plurality of implants comprising a shape memory material in a collapsed state,
b) delivering the plurality of implants through a cannula into the fractured vertebral body, and
c) expanding the plurality of implants to stabilize the fractured vertebral body.

The shape memory material may be a shape memory metal. The plurality of implants may have a collapsed shape selected from the group consisting of a sphere, a football, a coil, a cylinder, an ellipsoid, and a crumpled ball of wire. The plurality of implants may be sequentially inserted into the fractured vertebral body. The plurality of implants may be expanded through heat activated phase transformation. The plurality of implants may be expanded through superelastic deformation. The plurality of implants may be expanded to locally compact tissue and to create a network of small voids in the vertebral body. The method may further comprise the step of d) flowing a flowable material into the network of small voids. The flowable material may be selected from the group consisting of a bone cement and a bone growth agent. The flowable material may be a bone growth agent. The bone growth agent may comprise a growth factor. The bone growth agent may comprise a porous matrix. The bone growth agent may comprise viable cells. The method may further comprise the step of: d) lavaging the network of small voids.

In a further aspect of the invention, there is provided an intervertebral bone stent comprising:
a) a rod having a distal end portion, a proximal end portion and a threaded intermediate portion, and
b) a deformable shell having an upper wall, a lower wall, a distal intermediate wall located between the upper and lower walls, and a proximal threaded lumen
wherein the distal end portion of the rod is attached to the intermediate wall of the deformable shell, and
wherein the threaded intermediate portion of the rod is received in the threaded lumen.

In a further aspect of the invention, there is provided an intervertebral bone stent comprising:
a) a rod having a distal end portion forming a proximal shoulder, a proximal end portion having an enlarged head forming a distal shoulder, and a threaded intermediate shaft portion;
b) a threaded nut having a distal face, the nut threadably received upon the threaded intermediate shaft portion of the rod; and
c) a deformable shell having an upper wall and a lower wall, each wall having a proximal end and a distal end,
wherein the proximal end portion of each wall of the deformable shell bears against the distal face of the nut, and
wherein the distal end portion of each wall of the deformable shell bears against the proximal shoulder of the rod.

In a further aspect of the invention, there is provided an intervertebral bone stent comprising:
a) a tube having an outer surface, and inner threaded surface, a throughbore, and upper and lower slots extending from the outer surface to the throughbore, and a distal end shoulder radially extending from the outer surface;
b) a threaded nut having a distal face, the nut threadably received upon the threaded inner surface of the tube;
c) a plate having an upper end portion, a lower end portion, and an intermediate portion, the upper end of the plate extending from the upper slot and the lower end of the plate extending from the lower slot and
d) deformable upper and lower walls, each wall having a proximal end and a distal end,
wherein the distal face of the threaded nut abuts the intermediate portion of the plate, wherein the proximal end portion of the upper wall abuts the upper end portion of the plate,
wherein the proximal end portion of the lower wall abuts the lower end portion of the plate,
wherein the distal end portion of each wall abuts the distal end shoulder.

In a further aspect of the invention, there is provided an intervertebral bone stent comprising:
a) a rod having a distal end portion forming a proximal shoulder, an intermediate portion, and a proximal end portion,
b) a tube received upon the rod, the tube having an unslitted distal end and a plurality of intermediate longitudinal slits forming a plurality of collapsible walls having a distal end,
wherein the distal end portion of the rod extends from the tube, and
wherein the unslitted distal end of the tube bears against the proximal shoulder of the distal end portion of the rod.

In a further aspect of the invention, there is provided an intervertebral bone stent comprising:
a) a reversibly expanding structure containing multiple linkages capable of transitioning the structure from a collapsed shape to an expanded shape.

The reversibly expanding structure may have an inner void, and the stent may further comprise: b) a turnbuckle located within the inner void.

In a further aspect of the invention, there is provided an intravertebral stent, comprising:
a) a turnbuckle comprising a shaft having a first threaded end portion and a second oppositely threaded end portion,
b) a first nut threadably received upon the first threaded end portion,
c) a second nut threadably received upon the second oppositely threaded end portion,
d) an expandable structure comprising a plurality of struts and means for connecting the struts in a cooperative pattern, the struts including a first and second end struts,
wherein the first end strut bears against the first nut and the second end strut bears against the second nut.

In a further aspect of the invention, there is provided an intervertebral bone stent comprising:
a) a first hemi-tube having an inside surface, an outside surface and a first longitudinal hinge,
b) a second hemi-tube having an inside surface, an outside surface and a second longitudinal hinge, the inside surface of the second hemi-tube opposing the inside surface of the first hemi-tube to form an inner bore between the two hemi-tubes,
c) a cam located within the inner bore.

The cam may be substantially oval.

In a further aspect of the invention, there is provided an intravertebral bone stent comprising a tubular member comprising a shape memory material.

The shape memory material may have a martinsitic M→ austentic A phase change between 22 °C and 37 °C. The shape memory material may have a superelastic characteristic between 22 °C and 37 °C. The tubular member may be a mesh. The shape memory material may be selected from the group consisting of a metal and a polymer.

## Claims

1. An intervertebral bone stent comprising:
a) a reversibly expanding structure containing multiple linkages capable of transitioning the structure from a collapsed shape to an expanded shape, and an inner void; and
b) a membrane located within the inner void.

2. The stent of claim 1, wherein the structure is coupled with the membrane.

3. The stent of either of claims 1 or 2, wherein the membrane is a balloon.

4. The stent of any one of the preceding claims, wherein the membrane is adapted to expand the structure from the collapsed shape to the expanded shape and thus the stent from a closed position to an open position.

5. The stent of claim 4, wherein the membrane is a balloon and is adapted to be expanded from a collapsed state to an expanded state through the introduction of a sufficient amount of fluid into the balloon.

6. The stent of any one of the preceding claims, wherein an outer surface of the membrane is attached, in a collapsed state, to inner links of the multiple-linkage structure.

7. The stent of any one of claims 1 to 5, wherein an inner surface of the membrane is connected to outer links of the multiple-linkage structure.

8. The stent of any one of claims 1 to 3, 6 or 7, wherein the stent is adapted to be driven open to its expanded shape, thereby holding the membrane in a state of maximum volume, and enabling the void inside the membrane to be filled with a bone growth agent.

9. The stent of any one of the preceding claims, wherein the membrane, when in an expanded state, can be used to hold the stent open as a permanent part of the stent.

10. The stent of any one of claims 1 to 8, wherein the membrane is biodegradable.
